# EUROPEAN PATENT SPECIFICATION

(11) **EP 4 014 868 B1**
(45) Date of publication and mention of the grant of the patent: **11.03.2026**
(21) Application number: 20215019.9
(22) Date of filing: 17.12.2020
(51) Int. Cl.: A61B 5/352, A61B 5/16, A61B 5/00, G16H 50/20

(54) **COGNITIVE STATES**
KOGNITIVE ZUSTÄNDE
ÉTATS COGNITIFS

(43) Date of publication of application: 22.06.2022
(73) Proprietor: Nokia Technologies Oy, 02610 Espoo (FI)
(72) Inventor: MARASCU, Alice, Dublin 1 (IE); LINDHOLM, Harri, 00320 Helsinki (FI); SALA, Alessandra, Dublin 15 (IE)
(74) Representative: Whiting, Gary

(56) References cited:
- WO-A1-2020/053280
- US-A1- 2016 128 586
- US-A1- 2018 249 939
- AGRAFIOTI F ET AL: "ECG Pattern Analysis for Emotion Detection", IEEE TRANSACTIONS ON AFFECTIVE COMPUTING, IEEE, USA, vol. 3, no. 1, 1 January 2012 (2012-01-01), pages 102 - 115, XP011460780, ISSN: 1949-3045, DOI: 10.1109/T-AFFC.2011.28
- HAMADA RYUNOSUKE ET AL: "Real-time emotional state estimation system for Canines based on heart rate variability", 2017 IEEE INTERNATIONAL CONFERENCE ON CYBORG AND BIONIC SYSTEMS (CBS), IEEE, 17 October 2017 (2017-10-17), pages 298 - 303, XP033304957, DOI: 10.1109/CBS.2017.8266120
- GAETANO VALENZA ET AL: "Revealing Real-Time Emotional Responses: a Personalized Assessment based on Heartbeat Dynamics", SCIENTIFIC REPORTS, vol. 4, no. 1, 21 May 2014 (2014-05-21), XP055442213, DOI: 10.1038/srep04998
- "Method and System for Scheduling Calendar Events for Managing Stress of a User ED - Darl Kuhn", IP.COM, IP.COM INC., WEST HENRIETTA, NY, US, 10 April 2019 (2019-04-10), XP013182839, ISSN: 1533-0001

## Description

### Field

This present specification relates to human cognitive states.

### Background

Task performance of humans may vary based on their physical, mental, emotional, or cognitive state. There remains a need for further developments in determining and making use of cognitive states.

WO 2020/053280 describes a method for generating a consciousness indicator for a non-communicating subject. The method comprises: generating a sensory stimulation involve at least one of the five senses (said sensory stimulation comprising multiple consecutive stimuli including at least a first stimulus and a second stimulus relating to the same sense and different from one another); measuring an electrocardiographic signal of the subject during the generation of the sensory simulation; extracting at least one EKG feature from the electrocardiographic signal associated to cognitive processes; and generating a consciousness indicator using the EKG features as input of a classifier.

### Summary

The invention is as defined in the appended claims.

In a first aspect, this specification provides an apparatus comprising means for performing: receiving physiological measurements relating to a user, wherein the physiological measurements comprise heart beat measurements; determining, based on intervals between peaks of the physiological measurements, a first physiological pattern; determining a cognitive activation score based on a comparison between the first physiological pattern and a baseline physiological pattern of the user, wherein the baseline physiological pattern relates to a baseline state of the user and the first physiological pattern relates to a first emotional or cognitive state of the user.

In some examples, the cognitive activation score is determined based on one or more threshold differences between the first physiological pattern and the baseline physiological pattern.

In some examples, the heart beat measurements relate to electrocardiogram data of the user.

In some examples, the cognitive activation score comprises an indication of the user's emotional or cognitive state in relation to performing one or more tasks.

In some examples, the baseline physiological pattern is obtained based at least in part on inputs from the user when the user is subjected to one or more stimuli.

Some examples include means for: obtaining historical physiological patterns of the user; determining boundaries in the historical physiological pattern based on a plurality of emotional or cognitive states; and applying labels for the plurality of emotional or cognitive states.

In some examples, determining the first physiological pattern comprises: computing one or more minimum points and maximum points of received physiological measurements for a first threshold time period; and determining one or more R peaks using a peak detection function, wherein the peak detection function comprises detecting a first peak by performing: storing information for a first peak from a starting point of an increasing slope; computing a density of the first peak; computing a first angle of the first peak after the start of a decreasing slope; computing area of the first peak when the decreasing slope ends; if the starting point has a distance from a previous peak that is more than a threshold distance, then: computing alignment values of the first peak in relation to other peaks; and determining an whether the first peak is an R peak based on the alignment values.

Some examples include means for providing an indication of the cognitive activation score to the user and/or to one or more other users.

In some examples, the indication is provided in relation to one or more tasks performed or to be performed by the user.

Some examples include means for providing the indication on a calendar, wherein the indication is accompanied by time stamps on the calendar.

Some examples include means for scheduling one or more tasks based on the cognitive activation score of the user.

Some examples include means for sending a trigger to the user a first time period before the cognitive activation score of the user is predicted to be above or below a first threshold.

The means may comprise: at least one processor; and at least one memory including computer program code, the at least one memory and the computer program code configured, with the at least one processor, to cause the performance of the apparatus.

In a second aspect, this specification describes a method comprising: receiving physiological measurements relating to a user, wherein the physiological measurements comprise heart beat measurements; determining, based on intervals between peaks of the physiological measurements, a first physiological pattern; determining a cognitive activation score based on a comparison between the first physiological pattern and a baseline physiological pattern of the user, wherein the baseline physiological pattern relates to a baseline state of the user and the first physiological pattern relates to a first emotional or cognitive state of the user.

In some examples, the cognitive activation score is determined based on one or more threshold differences between the first physiological pattern and the baseline physiological pattern.

In some examples, the heart beat measurements relate to electrocardiogram data of the user.

In some examples, the cognitive activation score comprises an indication of the user's emotional or cognitive state in relation to performing one or more tasks.

In some examples, the baseline physiological pattern is obtained based at least in part on inputs from the user when the user is subjected to one or more stimuli.

Some examples may further comprise: obtaining historical physiological patterns of the user; determining boundaries in the historical physiological pattern based on a plurality of emotional or cognitive states; and applying labels for the plurality of emotional or cognitive states.

In some examples, determining the first physiological pattern comprises: computing one or more minimum points and maximum points of received physiological measurements for a first threshold time period; and determining one or more R peaks using a peak detection function, wherein the peak detection function comprises detecting a first peak by performing: storing information for a first peak from a starting point of an increasing slope; computing a density of the first peak; computing a first angle of the first peak after the start of a decreasing slope; computing area of the first peak when the decreasing slope ends; if the starting point has a distance from a previous peak that is more than a threshold distance, then: computing alignment values of the first peak in relation to other peaks; and determining an whether the first peak is an R peak based on the alignment values.

Some examples may further comprise: providing an indication of the cognitive activation score to the user and/or to one or more other users.

In some examples, the indication is provided in relation to one or more tasks performed or to be performed by the user.

Some examples may further comprise: providing the indication on a calendar, wherein the indication is accompanied by time stamps on the calendar.

Some examples may further comprise: scheduling one or more tasks based on the cognitive activation score of the user.

Some examples may further comprise: sending a trigger to the user a first time period before the cognitive activation score of the user is predicted to be above or below a first threshold.

In a third aspect, this specification describes an apparatus configured to perform any method as described with reference to the second aspect.

In a fourth aspect, this specification describes computer-readable instructions which, when executed by computing apparatus, cause the computing apparatus to perform any method as described with reference to the second aspect.

In a fifth aspect, this specification describes a computer program comprising instructions for causing an apparatus to perform at least the following: receiving physiological measurements relating to a user, wherein the physiological measurements comprise heart beat measurements; determining, based on intervals between peaks of the physiological measurements, a first physiological pattern; determining a cognitive activation score based on a comparison between the first physiological pattern and a baseline physiological pattern of the user, wherein the baseline physiological pattern relates to a baseline state of the user and the first physiological pattern relates to a first emotional or cognitive state of the user.

In a sixth aspect, this specification describes a computer-readable medium (such as a non-transitory computer-readable medium) comprising program instructions stored thereon for performing at least the following: receiving physiological measurements relating to a user, wherein the physiological measurements comprise heart beat measurements; determining, based on intervals between peaks of the physiological measurements, a first physiological pattern; determining a cognitive activation score based on a comparison between the first physiological pattern and a baseline physiological pattern of the user, wherein the baseline physiological pattern relates to a baseline state of the user and the first physiological pattern relates to a first emotional or cognitive state of the user.

In a seventh aspect, this specification describes an apparatus comprising: at least one processor; and at least one memory including computer program code which, when executed by the at least one processor, causes the apparatus to: receive physiological measurements relating to a user, wherein the physiological measurements comprise heart beat measurements; determine, based on intervals between peaks of the physiological measurements, a first physiological pattern; determine a cognitive activation score based on a comparison between the first physiological pattern and a baseline physiological pattern of the user, wherein the baseline physiological pattern relates to a baseline state of the user and the first physiological pattern relates to a first emotional or cognitive state of the user.

In an eighth aspect, this specification describes an apparatus comprising: a first module configured to receive physiological measurements relating to a user, wherein the physiological measurements comprise heart beat measurements; a second module for determining, based on intervals between peaks of the physiological measurements, a first physiological pattern; a third module for determining a cognitive activation score based on a comparison between the first physiological pattern and a baseline physiological pattern of the user, wherein the baseline physiological pattern relates to a baseline state of the user and the first physiological pattern relates to a first emotional or cognitive state of the user.

### Brief description of the drawings

Example embodiments will now be described, by way of example only, with reference to the following schematic drawings, in which:
FIG. 1 is a block diagram of a system in accordance with an example embodiment;
FIG. 2 is a block diagram of a system in accordance with an example embodiment;
FIG. 3 is a flow chart showing an algorithm in accordance with an example embodiment;
FIG. 4 is a block diagram of a system in accordance with an example embodiment;
FIG. 5 is a flow chart showing an algorithm in accordance with an example embodiment;
FIG. 6 is a block diagram of a system in accordance with an example embodiment;
FIG. 7 is a flow chart showing an algorithm in accordance with an example embodiment;
FIG. 8 is a flow chart showing an algorithm in accordance with an example embodiment;
FIG. 9 is a plot showing physiological measurements in accordance with an example embodiment;
FIG. 10 is a flow chart showing an algorithm in accordance with an example embodiment;
FIG. 11 is a flow chart showing an algorithm in accordance with an example embodiment;
FIG. 12 is a calendar in accordance with an example embodiment;
FIG. 13 is a block diagram of a system in accordance with an example embodiment;
FIG. 14 is a block diagram in accordance with an example embodiment;
FIG. 15 is a block diagram of components of a system in accordance with an example embodiment; and
FIGS. 16A and 16B show tangible media, respectively a removable non-volatile memory unit and a Compact Disc (CD) storing computer-readable code which when run by a computer perform operations according to example embodiments.

### Detailed description

The scope of protection sought for various embodiments of the invention is set out by the independent claims. The embodiments and features, if any, described in the specification that do not fall under the scope of the independent claims are to be interpreted as examples useful for understanding various embodiments of the invention.

In the description and drawings, like reference numerals refer to like elements throughout.

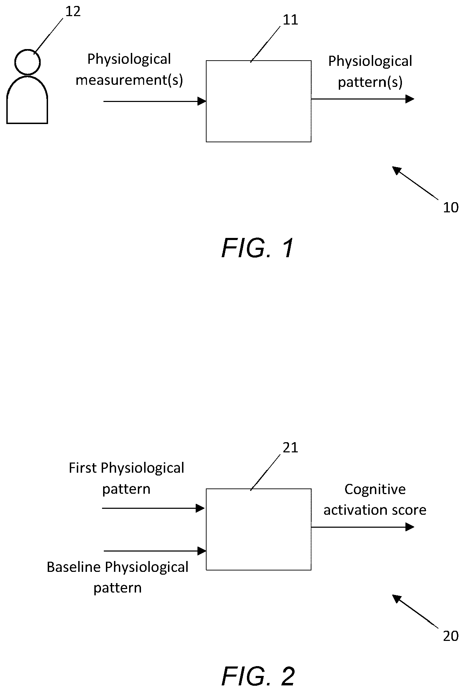

FIG. 1 is a block diagram of a system, indicated generally by the reference numeral 10, in accordance with an example embodiment. Physiological measurements relating to a user, such as user 12, may be received at a first module 11. The physiological measurements may comprise heart beat measurements, such as electrocardiography (ECG) data, of the user 12. The physiological measurements may be analysed at the first module 11 for determining physiological pattern(s). For example the physiological patterns may be determined based on peaks and/or patterns of peaks that are detected in the physiological data. In one example, the first module 11 may comprise a model for computing the physiological patterns based on the physiological measurements.

In one example, variations in physiological measurements may be indicators of variations in the state of humans (e.g. user 12). For example, variations of RR-intervals in heart signals may show less dense waves associated to relaxed states and dense waves to intense states. The example embodiments discussed herewith provide techniques for determining cognitive and/or emotional state of the user 12 by making use of the physiological measurements. The cognitive and/or emotional state of a human may be used for various purposes, such as determining and/or improving performance in tasks, improving effectiveness of assigning and/or scheduling tasks (e.g. avoiding accidents due to decisions or actions performed under stress or exhausted state) or the like.

FIG. 2 is a block diagram of a system, indicated generally by the reference numeral 20, in accordance with an example embodiment. System 20 comprises a second module 21. The second module 21 may receive a first physiological pattern, for example the physiological pattern relating to the user 12, as determined by the first module 11. The second module 21 is further provided with a baseline physiological pattern of the first user 12. The baseline physiological pattern of the first user 12 may relate to a baseline (e.g. a relaxed) state of the first user 12. For example, the baseline physiological pattern may be obtained from physiological measurements of the first user 12 when the first user 12 is in a relaxed state. Alternatively, or in addition, the baseline physiological pattern may be obtained from an external source, for example, based on averaged baseline datasets (e.g. medical averaged datasets used for providing medical consistency to the baseline datasets).

The second module 21 may be used for determining a cognitive activation score of the first user 12 based on a comparison between the first physiological pattern and the baseline physiological pattern of the first user. In one example embodiment, the first module 11 and the second module 21 may be comprised within a single module or apparatus.

In one example embodiment, the cognitive activation score may be indicative of the cognitive or emotional state of the user. For example, the larger the difference between the user's current state and baseline state, the more stressed the user may be deemed to be or the user may be required to have a higher cognitive activation state (e.g. for a difficult or challenging task). The cognitive activation score may be used for various purposes, such as monitoring of human cognitive performance, determining variations in state during a certain activity or time period, and/or extracting correlations with other factors that could potentially impact task performance (e.g., food intake, sleep, etc.). The cognitive activation score may also be used for collaborative task planning in teams. This is described in further detail below.

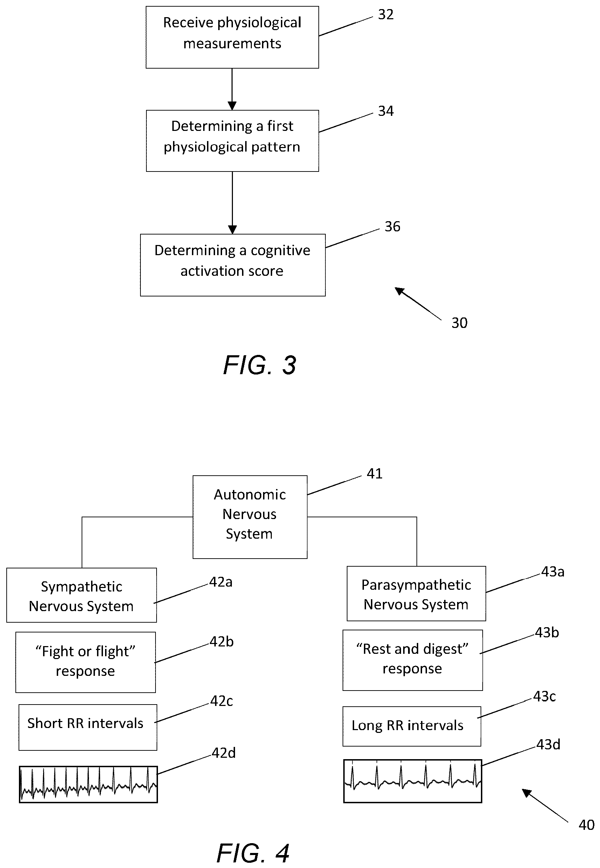

FIG. 3 is a flowchart of an algorithm, indicated generally by the reference numeral 30, in accordance with an example embodiment.

Algorithm 30 starts at operation 32, where physiological measurements relating to a user are received (e.g. at the first module 11). The physiological measurements may comprise heart beat measurements. The heartbeat measurements may, for example, relate to electrocardiography (ECG) data.

In an example embodiment, the physiological measurements may relate to data other than (or in addition to) heartbeat or ECG measurements. For example, the physiological measurements may relate to one or more of ballistocardiography (BCG) data, seismocardiography (SCG) data, photoplethysmography (PPG), gyrocardiography (GCG), and/or impedance cardiography (ICG) data. Alternatively, or in addition, the physiological measurements may relate to a breathing pattern. The skilled person will be aware of other physiological data that may be suitable for use in the algorithm 30.

At operation 34, a first physiological pattern is determined, for example, based on intervals between peaks of the physiological measurements. In an example embodiment, a machine learning model may be used for determining the first physiological pattern based on the peaks. For example, the forms or patterns of the peaks detected from the physiological measurements may be mined and extracted. For heartbeat measurements, for example, the R peaks may be detected, and the RR intervals used for determining the first physiological pattern.

In one example embodiment, if the physiological measurements relate to BCG data or electroencephalography (EEG) data, J peaks (relating to motion due to contractions during heart beats) may be detected rather than R peaks (relating to electrical activity triggered during heart beats). Alternatively, or in addition, if the physiological measurements relate to a breathing pattern, the first physiological pattern may be based on one or more shapes of the breathing pattern.

At operation 36, a cognitive activation score of the first user 12 may be determined based on a comparison between the first physiological pattern and a baseline physiological pattern of the first user 12. The baseline physiological pattern may relate to a baseline state of the user and the first physiological pattern may relate to a first emotional or cognitive state of the user. The first emotional or cognitive state of the user may relate to the user's state at the time of collecting the physiological measurements from the user.

FIG. 4 is a block diagram of a system, indicated generally by the reference numeral 40, in accordance with an example embodiment. System 40 shows a representation of the autonomic nervous system (ANS) 41 of a human. The autonomic nervous system 41 comprises a control system that acts largely unconsciously and regulates bodily functions, such as the heart rate, digestion, respiratory rate, or the like. The autonomic nervous system 41 may comprise a sympathetic nervous system 42a, a parasympathetic nervous system 43a, and an enteric nervous system (not shown).

The sympathetic nervous system 42a may relate to or promote a "fight or flight" response 42b, for example, corresponding with energy generation (e.g. opposite of a relaxed state). In such a state, for example, blood flow may be enhanced and heart rate of the user may increase. For example, the RR intervals 42c of ECG data shown in plot 42d may be relatively short RR intervals that may correspond to a stressed or energised state.

The parasympathetic nervous system 43a may relate to or promote a "rest and digest" response 43b, for example, corresponding to a relaxed state, thus promoting calming of the nerves. In such a state, for example, heart rate may be at or returned to a regular rate. For example, the RR intervals 43c of ECG data shown in plot 43d may be relatively long RR intervals that may correspond to a relaxed state. It can be seen from the comparison of plots 42d and 43d that the physiological measurements of a user may reflect whether the user is in relaxed state (long RR intervals) or a stressed or energised state (short RR intervals).

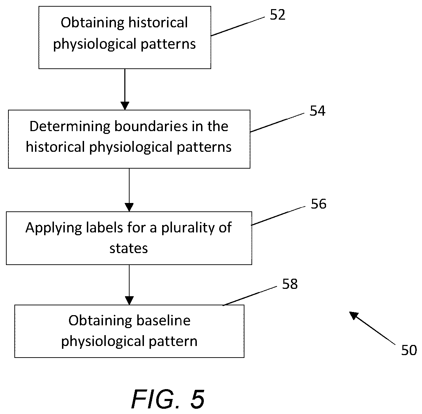

FIG. 5 is a flowchart of an algorithm, indicated generally by the reference numeral 50, in accordance with an example embodiment. The algorithm 50 may be used for obtaining a baseline physiological pattern of a user, such as the user 12, which baseline physiological pattern may relate to a baseline (e.g. relaxed) state of the user 12.

At operation 52, historical physiological patterns of the user 12 may be obtained. For example, the historical physiological patterns may be determined from historical physiological data collected from the user 12 at various time periods in the past. In one example embodiment, the historical physiological data may be used for inferring the baseline state of a user and/or intervals of variation in his/her cognitive activation scores under various cognitive or emotional states.

At operation 54, one or more boundaries are determined in the historical physiological patterns. For example, the one or more boundaries may define one or more respective points where a state ends and another state begins. The boundaries may be used for separating the different states of the plurality of historical physiological patterns and for computation of the respective cognitive activation score of the states.

At operation 56, one or more labels are applied to a plurality of emotional or cognitive states, for example as indicated by the boundaries. For example, a first label may indicate a relaxed state, a second label may indicate a positive emotional state, a third label may indicate a negative emotional state, a fourth label may indicate a low cognitive task state, a fifth label may indicate a high cognitive task state, and a sixth label may indicate self-evaluation state (e.g. when the user is evaluating their own emotional or cognitive state by answering questions or quizzes). Of course, other labels may be applied, in addition to, or instead of, at least some of the labels described above. For example, the labels may be known (e.g., by knowing the task the user was performing at that time, by self-labelling by the user) or unknown (e.g. the algorithm detects a separation of states and assigned labels to the states).

At operation 58, a baseline physiological pattern of the user 12 may be obtained based, at least in part, on the historical physiological patterns and the corresponding labels. The baseline physiological pattern may relate to a relaxed state of the user 12. It may be appreciated that different users may have different physiological patterns in a relaxed state, and therefore the historical physiological pattern of the specific user, such as user 12, may be used for determining the baseline physiological pattern of that specific user. Alternatively, or in addition, the baseline physiological pattern may be obtained from the user by allowing the user to go into a relaxed state (e.g. naturally or by stimulating relaxation) and collecting physiological data when the user is in a relaxed state.

In an example embodiment, the historical physiological pattern(s) may be obtained based at least in part on inputs from the user 12 when the user 12 is subjected to one or more stimuli. For example, the stimuli may be used for provoking certain states in the user 12, and physiological data of the user 12 may be collected while the user 12 is in the provoked states. In an example embodiment, a positive or negative emotional state may be provoked using visual stimulation (e.g. showing videos to the user); a low cognitive task state may be provoked using verbal talk stimulation; a high cognitive task state may be provoked using math or other complex problem stimulation; and self-evaluation state may be provoked in a self-evaluation phase, where the user may answer a set of questions extracting data on the user's general profile, for example, prior to the stimuli.

In one example, the stimulations for different states (e.g. positive emotional state and negative emotional state) are separated by a relaxation period, for example, of 90 seconds (medically known period of time for body relaxation). In one example, the self-evaluation questions may be based on psychometrics standards (e.g. taking user information prior to and/or during evaluation, number of questions, and answering scale format) and may include questions such as *"How are you feeling right now?", "How did you feel this morning?", "Where in your body do you feel tension now?".* For example, users may answer using a score (e.g. on a scale of 1 to 10), a drop-down menu (e.g. selecting body parts), or by entering the answer by typing. In one example, the problem stimulation for cognitive task state may include a test of backward digit span (e.g. reverse counting), or similar tasks. Of course, many alternatives to these examples are possible.

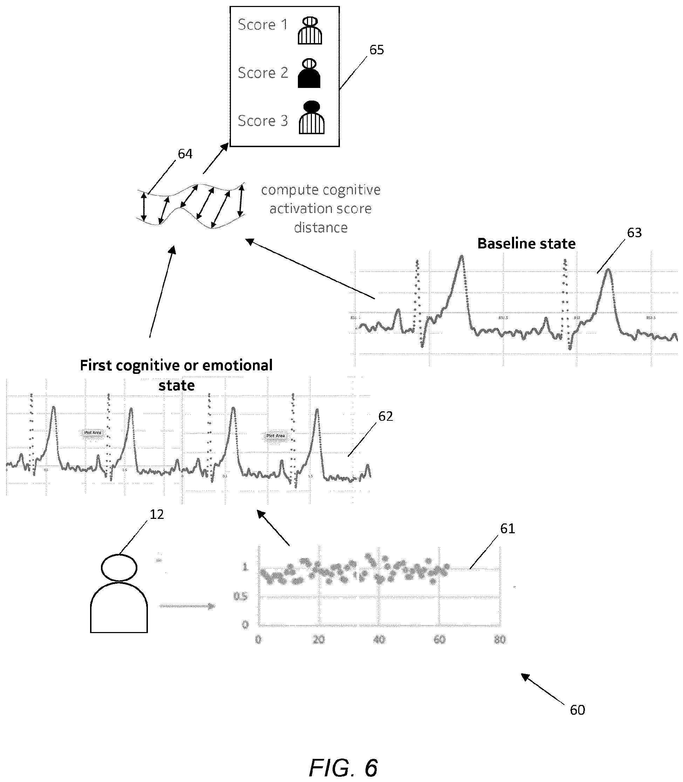

FIG. 6 is a block diagram of a system, indicated generally by the reference numeral 60, in accordance with an example embodiment. System 60 shows how a cognitive activation score can be determined from physiological data. Physiological measurements may be collected from user 12, and intervals between peaks (e.g. RR intervals) of the physiological measurements may be determined, as shown in the plot 61. A first physiological pattern, as shown in plot 62, may be determined based on the intervals between the peaks. The first physiological pattern may relate to a first cognitive or emotional state of the user 12. The baseline state of the user 12, as shown in plot 63, may be obtained (e.g. determined and stored/obtained previously) in order to perform a comparison with the first cognitive or emotional state. The distance (difference) between the plots 62 and 63 may be calculated, as shown in plot 64, in order to determine the cognitive activation score. The cognitive activation score may be provided, for example, as shown in block 65.

In one example, the cognitive activation score is determined based on one or more threshold differences between the first physiological pattern and the baseline physiological pattern. For example, if the distance between the first physiological pattern and the baseline physiological pattern is less than a first threshold difference, then the cognitive activation score may be determined to be a first score (score 1, shown by a dotted figure); if the distance between the first physiological pattern and the baseline physiological pattern is higher than the first threshold difference but less than a second threshold difference, then the cognitive activation score may be determined to be a second score (score 2, shown by vertically striped figure); if the distance between the first physiological pattern and the baseline physiological pattern is higher than the second threshold difference, then the cognitive activation score is determined to be a third score (score 3, shown by a horizontally striped figure). For example, the first score may represent a relaxed state, the second score may represent a medium state of cognitive activation, and the third score may represent a high cognitive activation state. It would be appreciated that when the difference with the baseline relaxed state is higher, the user is likely to be more cognitively activated (e.g. in some cases that can mean more stressed).

In an example embodiment, the cognitive activation score may be obtained as a numerical value between 0 and 1, with 0 meaning no difference of activation between the states and 1 being highest difference between the states. Using a mathematical function to transform the interval [0,1] values into a discrete set of values allows easier usage in real life applications. One example is to use a discrete transformation into three equidistant bins, thus the values between [0, 1/3] would be in a first category (bin), the ones from [1/3, 2/3] would be in a second category (bin) and [2/3, 1] would be in a third category (bin). This transformation may be used as an illustration in one example, where the first category may indicate a low cognitive activation (score 1, shown by a dotted figure), the second category may indicate a medium cognitive activation (score 2, shown by vertically striped figure), and the third category may indicate a high cognitive activation (score 3, shown by a horizontally striped figure).

It would be appreciated that fewer or more than three categories may be identified by defining fewer or more threshold differences. For example, the score levels may include more than three scores (e.g. four or more), and this may provide more granularity in the determination of the score.

In one example, the distance (difference) between the first physiological pattern (e.g. the pattern 62) and the baseline physiological pattern (e.g. the pattern 63) may be determined as the cost of transforming the relaxed state time series (baseline physiological pattern) into another state's time series (first physiological pattern).

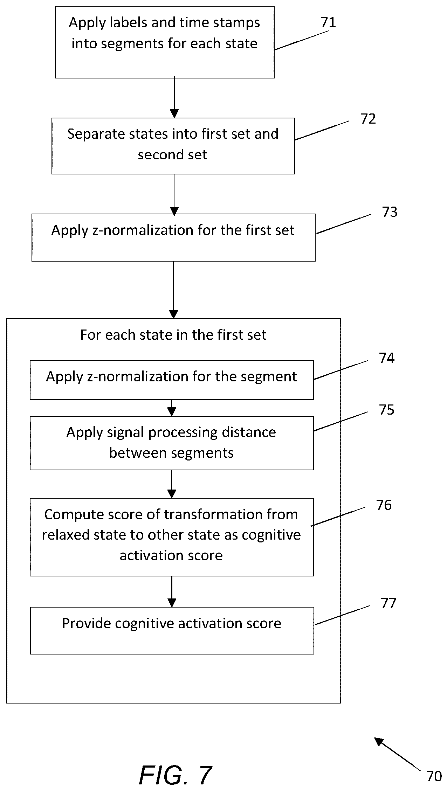

FIG. 7 is a flowchart of an algorithm, indicated generally by the reference numeral 70, in accordance with an example embodiment. Algorithm 70 may be described in conjunction with system 60. The physiological measurements from the user 12 may be received as a streaming time series (e.g. corresponding to a bio-signal, such as ECG data), and the first physiological pattern (e.g. sequence of timely R peaks), as shown in plot 62, is obtained. The algorithm 70 may then be used for determining the cognitive activation score.

Algorithm 70 starts at operation 71, where one or more labels (e.g. obtained earlier when determining baseline physiological pattern, as described in FIG. 5), are applied to the first physiological pattern. The labels are applied to separate each state into segments, and time stamps are also applied to said segments (e.g. time stamps indicating start and end of each state).

At operation 72, the plurality of states (segments) are separated into a first set and a second set. The first set includes the segment relating to a relaxed state, and the second set includes segments relating to all other states.

At operation 73, mathematical operations of rescaling (e.g. z-normalization) are applied to the time series of the segment relating to the relaxed state in the first set.

For at least one or each of the segments in the second set, operations 74 to 77 are performed (e.g. performed separately for each segment). For example, for a first segment of the second set, at operation 74, z-normalization is applied to the time series of the first segment. At operation 75, signal processing distance is applied between at least two segments of the time series (e.g. dynamic time warping distance, or based on the longest common subsequence). At operation 76, a score of transformation from a relaxed state to a state corresponding to the first segment is computed, where the score of transformation is determined to be the cognitive activation score. At operation 77, the cognitive activation score may be provided to a user, for example, as shown in block 65 (e.g. as a numerical value or transformed into a set of labels corresponding to a discrete transformation, e.g. into the first, second or third categories ). The cognitive activation score may therefore be provided for each of the states in the second set (e.g. such that the cognitive activation score may be used for diverse applications).

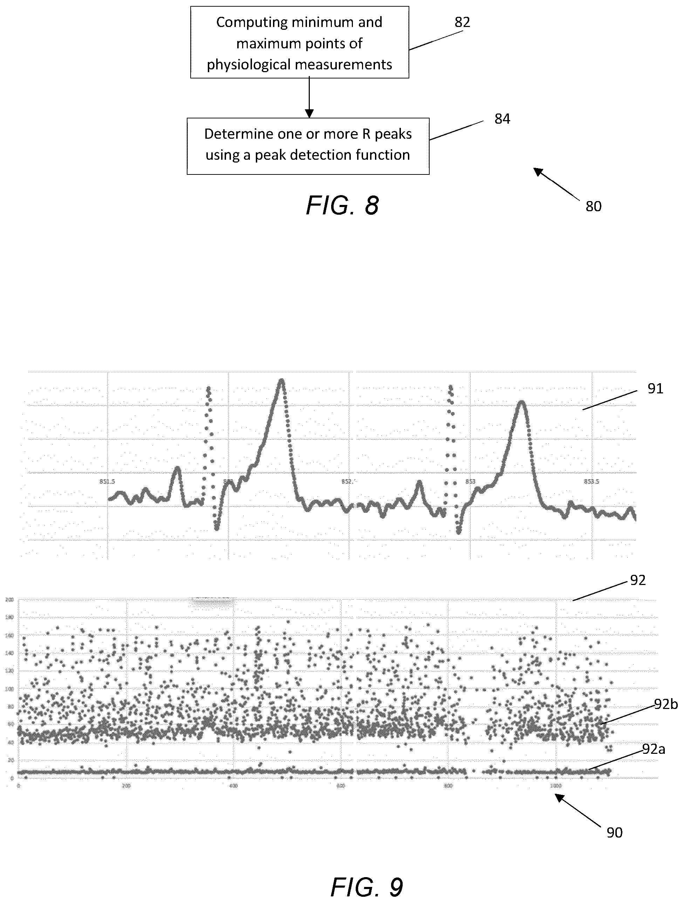

FIG. 8 is a flowchart of an algorithm, indicated generally by the reference numeral 80, in accordance with an example embodiment. The algorithm 80 may be used for determining on or more R peaks of physiological measurements, such as ECG data. The intervals between the R peaks may then be used for determining the physiological patterns.

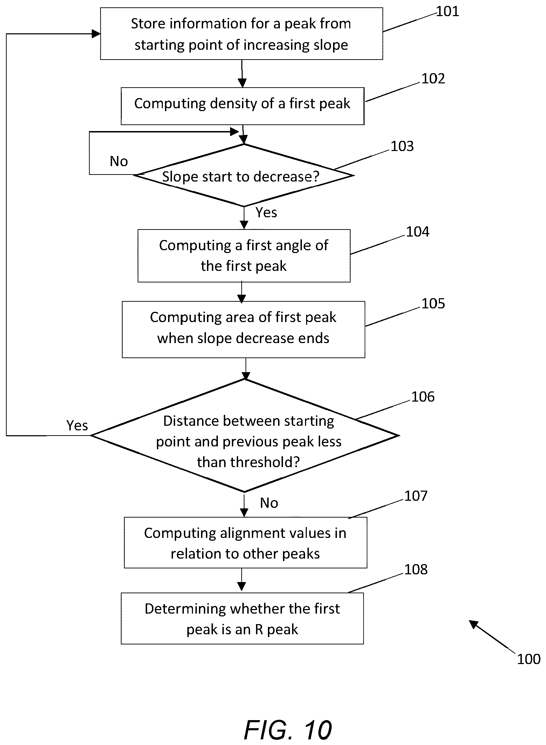

At operation 82, minimum and maximum points of a time series of the physiological measurements may be determined for a first threshold time period (e.g. while time is less than 3 seconds). At operation 84, one or more R peaks may be determined using a peak detection function. The peak detection function is described in further detail below with reference to FIG. 10.

FIG. 9 is a plot 90 showing physiological measurements in accordance with an example embodiment. The plot 91 may represent input data, such as the physiological measurements of a user, such as the user 12. For example, the plot 91 comprises ECG data related to the heart beat of the user 12. Plot 92 shows peak data separation of the physiological measurements of plot 91. For example, the data may be separated into two clouds, such that the lower cloud 92a may represent the extracted R peaks data, and the upper clouds 92b may represent other peaks data. The extracted R peaks may be determined using a peak detection function, for example, at operation 84. The peak detection function is described in further detail below with reference to FIG. 10.

FIG. 10 is a flowchart of an algorithm, indicated generally by the reference numeral 100, in accordance with an example embodiment. The algorithm 100 may be used in the peak detection function for determining R peaks of the physiological measurements. The algorithm 100 may start from a starting point of an increasing slope, such that when an increase in the slope of the physiological measurements is first detected, algorithm 100 may be started. If an increase in the slope is not detected at a point, then that point is skipped, and the next point is considered.

At operation 101, information for a first peak is stored from a starting point of the increasing slope. The starting point of the increasing slope may be considered to be the start of the peak formation of the first peak. The information may include information of the data points comprised within the first peak. Operation 101 may be performed for one or more peaks. In an example, a gap distance (e.g. based on medical knowledge on minimum distances possible) between each point of the first peak and a previous peak may be determined, and if the gap distance is lower than a threshold gap distance (e.g. 0.5 seconds), then the point may be discarded as being a point leading to a peak formation (e.g. function for a peak formation).

At operation 102, a density of the first peak is determined (e.g. based on medical knowledge on that signal). The density of the peak may be computed as the number of points per upward and downward shape of the first peak. For example, R peaks may have a smaller density compared to T peaks, such that the density value may be used for discretization. In one example, operations 101 and 102 may be performed for each point of the first peak where the slope is detected to be increasing.

If it is detected, at operation 103, that the slope has started to decrease, then a first angle of the first peak (e.g. angle formed by starting point, top peak point, and point in the downhill slope) is computed at operation 104. The first angle may be a peak angle that may be used for discrimination of R peak among other peaks. In one example, the R peaks may be determined, at least in part, based on medical knowledge on the signal shapes.

At operation 105, an area of the first peak may be computed when the slope decrease ends (i.e. area formed by the starting point of the peak, the top hill point, and the ending point of the downward slope).

If it is determined, at operation 106, that the distance between the starting point of the first peak and a previous peak is less than a threshold distance, then the first peak is discarded and algorithm 101 is started for a next starting point (e.g. of a second peak).

If it is determined, at operation 106, that the distance between the starting point of the first peak and a previous peak is higher than the threshold distance, then at operation 107, alignment values of the first peak may be computed. The alignment values may comprise geometrical alignment values (e.g. starting point and ending point positions compared to that of other peaks). The alignment values may be used for assigning a label (e.g. PQRST label) to the peak.

At operation 108, it is determined, based on the label, whether the first peak is an R peak. For example, geometrical distance scores (comparing the first peak with other peaks) may be used for checking whether the first peak is an R peak.

In one example, an output of the algorithm 100 comprises a set of all R peaks data (or the lower cloud data, as illustrated in plot 92) along with temporal and geometrical distances between them. For example, these chains of data may form physiological patterns, as described above.

In one example, the following pseudocode may be used for the algorithms 80 and 100:

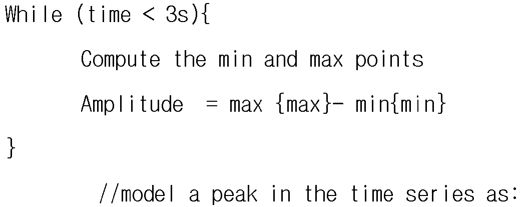

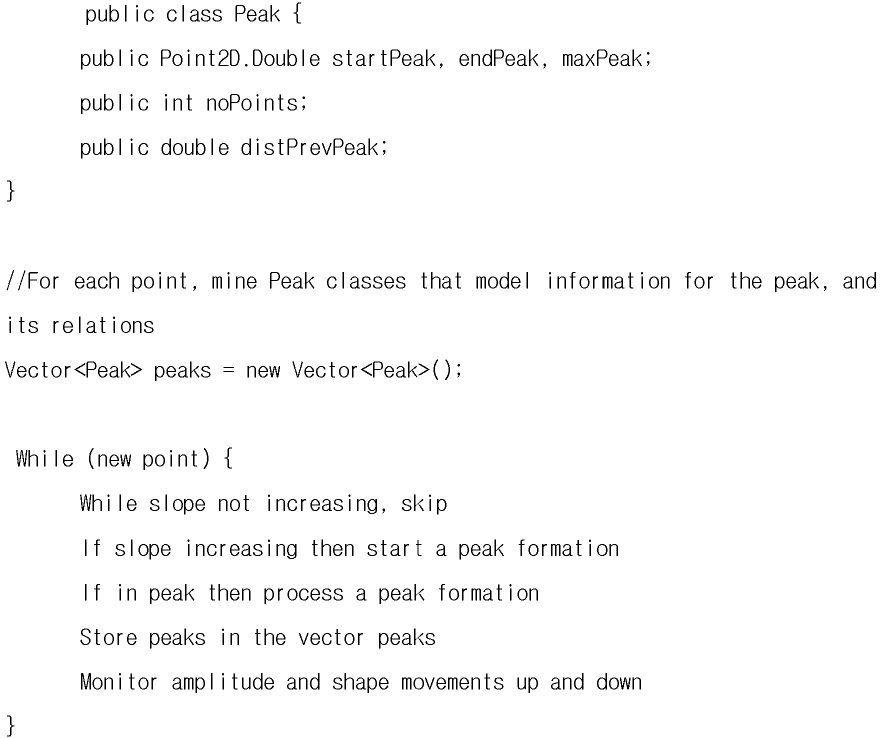

In an example embodiment, the cognitive activation score, for example, as determined in operation 36 with reference to FIG. 3, may comprise an indication of the emotional or cognitive state of the user (e.g. the user 12) in relation to performing one or more tasks. For example, the user's cognitive state may be determined at the time of performing one or more tasks, such that the user's task performance or efficiency may be determined based on the emotional or cognitive state. Consequently, one or more tasks to be performed by the user may also be scheduled such that the user is in a high cognitive state or a positive emotional state when performing the tasks. Alternatively, or in addition, if a task is a group task, or one or more people can perform the same task, the cognitive state of the one or more people may be used for assigning the task to one of the people by determining who is in the best state to perform the task.

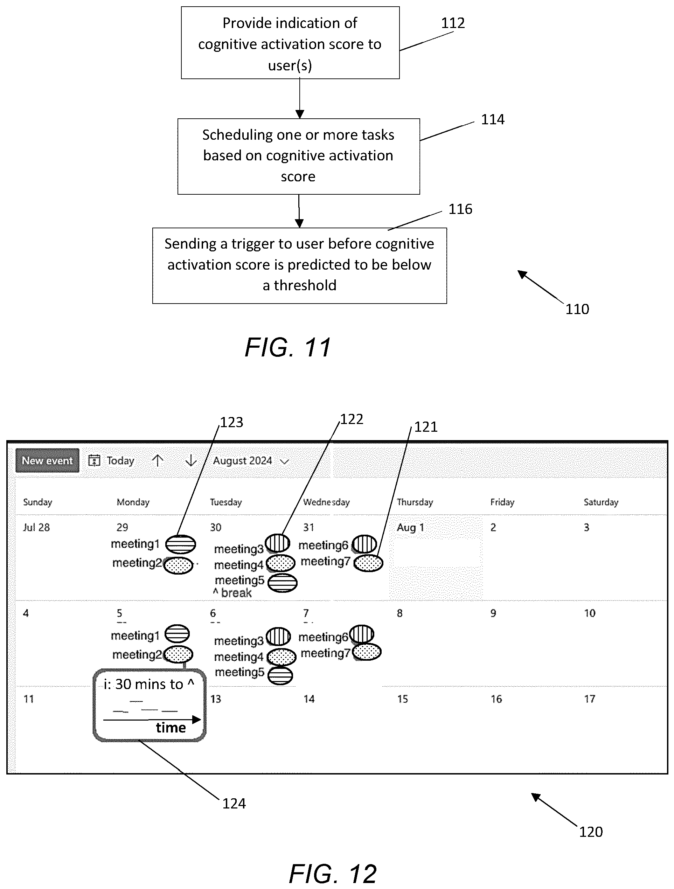

FIG. 11 is a flowchart of an algorithm, indicated generally by the reference numeral 110, in accordance with an example embodiment. The operations of the algorithm 110 may be performed independently, such that they may not need to follow any specific sequence or require any of the other operations to be performed.

At operation 112, an indication of the cognitive activation score is provided to the user and/or to one or more other users. For example, the indication may comprise a visual indication, audio indication, or any other indication for allowing the user or one or more other users to know the cognitive activation score of the user corresponding to a specific time period(s). For example, the indication may be provided as shown in block 65 with reference to FIG. 6, such that the cognitive activation score may be a first score, second score, or third score. In one example embodiment, the indication of the cognitive activation score of user 12 may be provided to one or more other users in situations where the user 12 works with (e.g. supervised by) the other users or has one or more group tasks with the other users. For example, the cognitive activation score of the user 12 may be used for assigning tasks to the user 12 and/or the other users.

At operation 114, one or more tasks of the user 12 may be scheduled based on the cognitive activation score of the user 12. For example, decisions regarding the task scheduling may be made according to the physiological pattern. As discussed above, the physiological pattern may be determined using a model (e.g. a physiological rhyme model) based on machine learning techniques. The model may further be used for determining the cognitive activation score based on distances with a baseline pattern (e.g. using the relaxed human state pattern as baseline for the computation of variations in other states).

At operation 116, one or more triggers may be sent to the user a first time period before the cognitive activation score of the user is predicted to be above or below a first threshold. Cognitive activation score of the user for different times of the day may be predicted based on machine learning techniques. For example, the user's data may be collected over a period of time to train a model, such that it can be predicted that a user is relaxed at certain times of the day or in response to certain events (e.g. massage, lunch, etc.), or that a user may be stressed at certain time of the day or in response to certain events (e.g. high workload, after work meeting, before doctor's appointment, etc.). As such, a trigger may be sent to the user if the cognitive activation score is predicted to be above or below a first threshold, where the cognitive activation score being above or below the first threshold may indicate whether the user is predicted to be in a relaxed state or stressed state. For example, if a trigger is sent to the user, the user may be reminded that the user is predicted to be in a stressed state after the first time period. The user may then be able to decide their actions or understand their cognitive activation state accordingly.

FIG. 12 comprises a calendar, indicated generally by the reference numeral 120, in accordance with an example embodiment. The indication of cognitive activation score(s) of a user, such as the user 12, may be provided on the calendar 120. In one example, the indications may be accompanied by time stamps on the calendar, such that the date and time corresponding to cognitive states can be seen from the calendar.

As shown in the calendar 120, for the dates of 29 July 2024 to 10 August 2024, user 12 may be scheduled to have meetings 1 and 2 on Mondays, meetings 3, 4, and 5 on Tuesdays, and meetings 6 and 7 on Wednesdays. In this example, three cognitive activation scores (e.g. illustrated in block 65 with reference to FIG. 6) are shown, including a first score 121 (e.g. representing a relaxed state) indicated by a dotted circle, a second score 122 (e.g. representing a medium state) indicated by a vertically striped circle, and a third score 123 (e.g. representing a stressed state) indicated by a horizontally striped circle. As such, cognitive activation scores for meetings 2, 4, and 7 are shown to be the first score (dotted circle), cognitive activation scores for meetings 3 and 6 are shown to be the second score (vertically striped circle), and cognitive activation scores for meeting 1 and 5 are shown to be the third score (horizontally striped circle).

In one example, for time periods where the cognitive activation score is shown or predicted to be the third score (e.g. stressed state), a recommendation for taking a break may be added to the calendar. For example, on 30^{th} July, Tuesday, a break is recommended after meeting 5, as meeting 5 is shown to have the third score.

In one example, the indications of cognitive activation score may be predicted based on the nature of the meetings, the time of the day, one or more other tasks or meetings the user may have, or the like.

In one example, an information item 124 may be provided to the user for example, for a time period (e.g. specific time period requested) to show the cognitive activation score through that time period. For example, the plot in item 124 shows the cognitive activation scores with respect to time. The item 124 may further include information regarding the cognitive activation score after a first time period (e.g. 30 minutes later), thus informing the user that the user may have a certain state (relaxed or stressed) after the first time period.

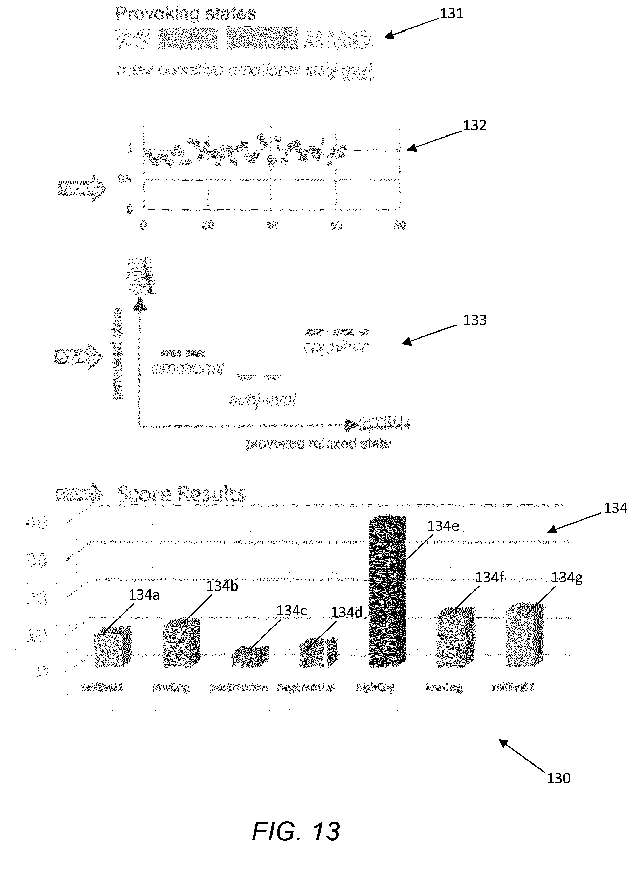

FIG. 13 is a block diagram of a system, indicated generally by the reference numeral 130, in accordance with an example embodiment. System 130 shows stages for determining average cognitive activation scores and emotional or cognitive states indicated by certain cognitive activation scores. These stages may be applied to a single user, or a group of users for obtaining average values. At a first stage 131, states, such as relaxed state, high or low cognitive state, positive or negative emotional state, or self-evaluation state, are provoked by presenting stimuli to the user(s). At stage 132, peak detection functions may be used to determine physiological patterns corresponding to each of the provoked states. At stage 133, each of the cognitive, emotional, or self-evaluation states are compared against the relaxed state, thus obtaining differences in physiological patterns for the corresponding states and the baseline state. At stage 134, score results of the cognitive activation score (vertical axis) may be determined (e.g. based on the difference with relaxed state), thus indicating which scores relate to which emotional or cognitive state (horizontal axis). For example, a first self-evaluation state 134a is indicated by cognitive activation score of around 8, a first low cognitive state 134b is indicated by cognitive activation score of around 9, a positive emotional state 134c is indicated by cognitive activation score of around 2, a negative emotional state 134d is indicated by cognitive activation score of around 5, a high cognitive state 134e is indicated by cognitive activation score of around 38, a second low cognitive state 134f is indicated by cognitive activation score of around 11, and a second self-evaluation state 134g is indicated by cognitive activation score of around 12. Note that these scores may be approximate scores (for individuals or averaged), and may vary from person to person.

In one example implementation of the principles described herein, a stimuli provided to the user (e.g. for provoking states) includes audio and/or video stimuli to provoke different states in the user. For example, the stimuli may be provided by means of a speaker and/or screen. A test scenario is composed of a sequence of timed states that presented to the user. There may be three types of states: information presentation to the user (video/audio), demand of input from the user (audio/visual, question answering, self-evaluation answered orally by the subject and registered in the form by the controller person), and relaxation of the subject between the states. For all self-evaluation questions, the user is asked to answer using a scale of values e.g. of [-2, -1, 0, 1, 2] with -2 being the lowest value, zero neutral, and +2 the highest value. The transition between the different states may be done through relaxation periods of 90 seconds (90 seconds may be the minimum duration needed for the heart rate variability constraints). No stimuli is used during the relaxation periods.

For example, first, the sequence of states may comprise a relaxation phase as an initial phase to help the user disconnect from their previous activities and focus on the present task. The relaxation may consist of watching a video, such as a relaxing video that was medically tested in the medical literature. Second, the sequence of states may further comprise a subject self-evaluation phase (134a), where the user answers (e.g. orally) a set of questions presented on the screen extracting data on subject's general profile, subject's state prior to experience. Third, the sequence of states may further comprise a first cognitive test (134b) of backward digit span to be solved by the user. Fourth, the sequence of states may further comprise a phase of inducing emotional states using visual triggers. One positive (134c) and one negative (134d) emotional state phase may be used, which may be separated by a relaxation period of 90 seconds. Fifth, a second cognitive test (134e and/or 134f) of backward digit span to be solved by the user. Sixth, a second subject self-evaluation phase (134g), where the user answers (e.g. orally) to a set of questions that were presented on the screen extracting data on subject's state post to the experience.

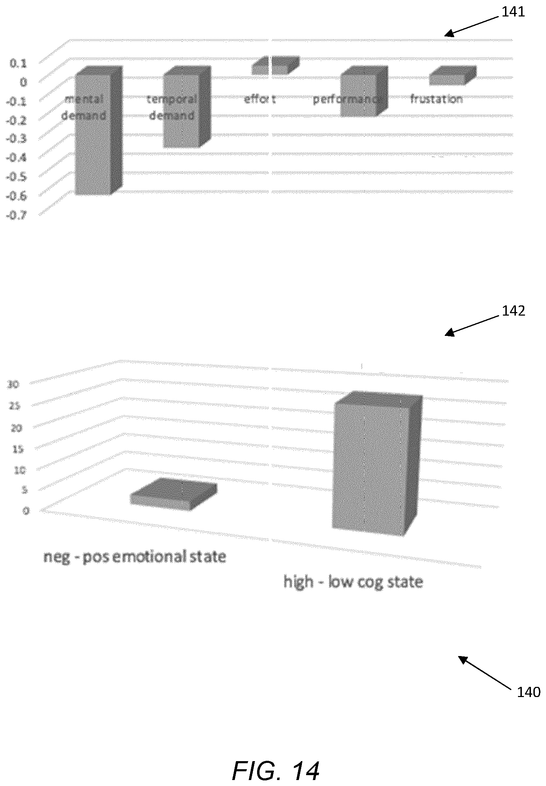

FIG. 14 is a block diagram of experiment results, indicated generally by the reference numeral 140, in accordance with an example embodiment. Distances between different cognitive states (more complex cognitive task versus lower cognitive task) have been computed, and a higher score is identified in the more complex tasks when compared to the lower complex tasks. Therefore, the cognitive activation score (e.g. as determined in operation 36 with reference to FIG. 3) can be used for determining and/or monitoring variations in the cognitive level of activities. This is shown in results 141 indicating correlation patterns between high cognitive state and a five dimensional vector of NASA-TLX (the five dimensions including mental demand, temporal demand, effort, performance, and frustration). Cognitive activation score computed based on physiological patterns is found to be higher when the user perceives higher mental and temporal demands compared to that in a relaxed state.

The cognitive activation score may also identify variations between the cognitive activation in negative emotional states compared to positive emotional states. Results 142 show captured amplitude of cognitive activation scores for negative and positive emotional states and high or low cognitive states. This shows that the cognitive effort for negative or positive emotional state is significantly less compared to that of high or low cognitive states.

Correlations between the data in self-evaluation forms were also computed. This illustrated the comparison between the user's subjective self-evaluation and states reflected by the physiological data (computed cognitive activation score).

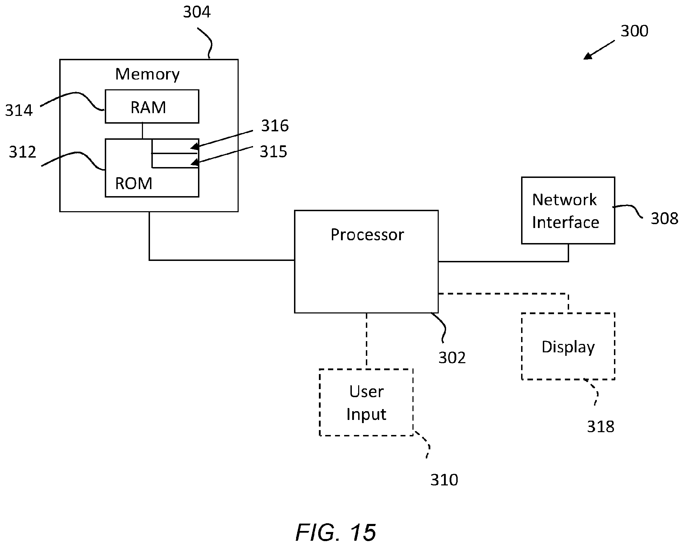

For completeness, FIG. 15 is a schematic diagram of components of one or more of the example embodiments described previously, which hereafter are referred to generically as a processing system 300. The processing system 300 may, for example, be the apparatus referred to in the claims below.

The processing system 300 may have a processor 302, a memory 304 closely coupled to the processor and comprised of a RAM 314 and a ROM 312, and, optionally, a user input 310 and a display 318. The processing system 300 may comprise one or more network/ apparatus interfaces 308 for connection to a network/ apparatus, e.g. a modem which may be wired or wireless. The interface 308 may also operate as a connection to other apparatus such as device/apparatus which is not network side apparatus. Thus, direct connection between devices/apparatus without network participation is possible.

The processor 302 is connected to each of the other components in order to control operation thereof.

The memory 304 may comprise a non-volatile memory, such as a hard disk drive (HDD) or a solid state drive (SSD). The ROM 312 of the memory 304 stores, amongst other things, an operating system 315 and may store software applications 316. The RAM 314 of the memory 304 is used by the processor 302 for the temporary storage of data. The operating system 315 may contain code which, when executed by the processor implements aspects of the algorithms 30, 50, 70, 80, 100, and 110 described above. Note that in the case of small device/apparatus the memory can be most suitable for small size usage i.e. not always a hard disk drive (HDD) or a solid state drive (SSD) is used.

The processor 302 may take any suitable form. For instance, it may be a microcontroller, a plurality of microcontrollers, a processor, or a plurality of processors.

The processing system 300 may be a standalone computer, a server, a console, or a network thereof. The processing system 300 and needed structural parts may be all inside device/apparatus such as IoT device/apparatus i.e. embedded to very small size.

In some example embodiments, the processing system 300 may also be associated with external software applications. These may be applications stored on a remote server device/apparatus and may run partly or exclusively on the remote server device/apparatus. These applications may be termed cloud-hosted applications. The processing system 300 may be in communication with the remote server device/apparatus in order to utilize the software application stored there.

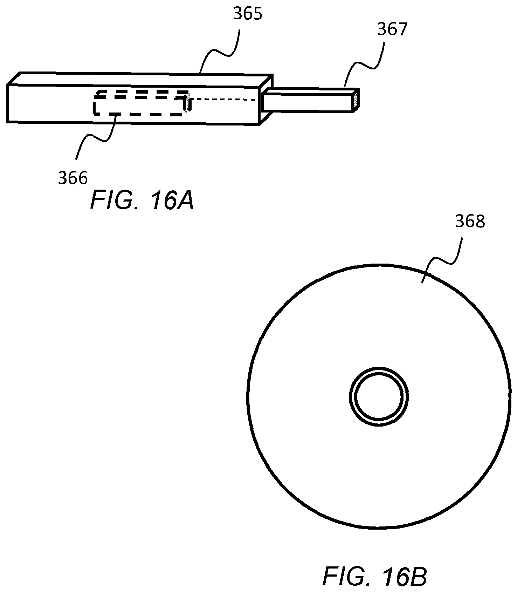

FIGS. 16A and 16B show tangible media, respectively a removable memory unit 365 and a compact disc (CD) 368, storing computer-readable code which when run by a computer may perform methods according to example embodiments described above. The removable memory unit 365 may be a memory stick, e.g. a USB memory stick, having internal memory 366 storing the computer-readable code. The internal memory 366 may be accessed by a computer system via a connector 367. The CD 368 may be a CD-ROM or a DVD or similar. Other forms of tangible storage media may be used. Tangible media can be any device/ apparatus capable of storing data/information which data/information can be exchanged between devices/apparatus/network.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices/apparatus and other devices/apparatus. References to computer program, instructions, code etc. should be understood to express software for a programmable processor firmware such as the programmable content of a hardware device/apparatus as instructions for a processor or configured or configuration settings for a fixed function device/apparatus, gate array, programmable logic device/apparatus, etc.

If desired, the different functions discussed herein may be performed in a different order and/or concurrently with each other. Furthermore, if desired, one or more of the above-described functions may be optional or may be combined. Similarly, it will also be appreciated that the flow diagrams of Figures 3, 5, 7, 8, 10, and 11 are examples only and that various operations depicted therein may be omitted, reordered and/or combined.

It will be appreciated that the above described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

Although various aspects of the invention are set out in the independent claims, other aspects of the invention comprise other combinations of features from the described example embodiments and/or the dependent claims with the features of the independent claims, and not solely the combinations explicitly set out in the claims.

It is also noted herein that while the above describes various examples, these descriptions should not be viewed in a limiting sense. Rather, there are several variations and modifications which may be made without departing from the scope of the present invention as defined in the appended claims.

## Claims

1. An apparatus comprising means for performing:
receiving (32) physiological measurements relating to a user, wherein the physiological measurements comprise heart beat measurements;
determining (34), based on intervals between peaks of the physiological measurements, a first physiological pattern;
determining (36) a cognitive activation score based on a comparison between the first physiological pattern and a baseline physiological pattern of the user, wherein the baseline physiological pattern relates to a baseline state of the user and the first physiological pattern relates to a first emotional or cognitive state of the user;
**characterized by**
providing (112) a predicted indication of cognitive activation score to the user and/or to one or more other users, wherein the predicted indication is provided in relation to one or more tasks performed or to be performed by the user, and wherein the predicted indication of cognitive activation score is generated based, at least in part, on the nature of the one or more tasks, using a model created from collected user data.

2. An apparatus as claimed in claim 1, wherein the cognitive activation score is determined based on one or more threshold differences between the first physiological pattern and the baseline physiological pattern.

3. An apparatus as claimed in any one of the preceding claims, wherein the heart beat measurements relate to electrocardiogram data of the user.

4. An apparatus as claimed in any one of the preceding claims, wherein the cognitive activation score comprises an indication of the user's emotional or cognitive state in relation to performing one or more tasks.

5. An apparatus as claimed in any one of the preceding claims, wherein the baseline physiological pattern is obtained based at least in part on inputs from the user when the user is subjected to one or more stimuli.

6. An apparatus as claimed in claim 5, further comprising means for:
obtaining (52) historical physiological patterns of the user;
determining (54) boundaries in the historical physiological pattern based on a plurality of emotional or cognitive states; and
applying (56) labels for the plurality of emotional or cognitive states.

7. An apparatus as claimed in any one of the preceding claims, wherein determining the first physiological pattern comprises:
computing (82) one or more minimum points and maximum points of received physiological measurements for a first threshold time period; and
determining (84) one or more R peaks using a peak detection function,
wherein the peak detection function comprises detecting a first peak by performing:
storing (101) information for a first peak from a starting point of an increasing slope;
computing (102) a density of the first peak;
computing (104) a first angle of the first peak after the start of a decreasing slope;
computing (105) area of the first peak when the decreasing slope ends;
if the starting point has a distance from a previous peak that is more than a threshold distance, then:
computing (107) alignment values of the first peak in relation to other peaks; and
determining (108) an whether the first peak is an R peak based on the alignment values.

8. An apparatus as claimed in any of the preceding claims, further comprising means for providing the indication on a calendar, wherein the indication is accompanied by time stamps on the calendar.

9. An apparatus as claimed in any of the preceding claims, further comprising means for scheduling (114) one or more tasks based on the cognitive activation score of the user.

10. An apparatus as claimed in any one of the preceding claims, further comprising means for sending (116) a trigger to the user a first time period before the cognitive activation score of the user is predicted to be above or below a first threshold.

11. A method comprising:
receiving (32) physiological measurements relating to a user, wherein the physiological measurements comprise heart beat measurements;
determining (34), based on intervals between peaks of the physiological measurements, a first physiological pattern;
determining (36) a cognitive activation score based on a comparison between the first physiological pattern and a baseline physiological pattern of the user, wherein the baseline physiological pattern relates to a baseline state of the user and the first physiological pattern relates to a first emotional or cognitive state of the user;
**characterized by**
providing (112) a predicted indication of cognitive activation score to the user and/or to one or more other users, wherein the predicted indication is provided in relation to one or more tasks performed or to be performed by the user, and wherein the predicted indication of cognitive activation score is generated based, at least in part, on the nature of the one or more tasks using a model created from collected user data.

12. A method as claimed in claim 11, wherein the cognitive activation score is determined based on one or more threshold differences between the first physiological pattern and the baseline physiological pattern.

13. A computer program comprising instructions which, when the program is executed by a computer, cause the computer to perform at least the following:
receiving physiological measurements relating to a user, wherein the physiological measurements comprise heart beat measurements;
determining, based on intervals between peaks of the physiological measurements, a first physiological pattern;
determining a cognitive activation score based on a comparison between the first physiological pattern and a baseline physiological pattern of the user, wherein the baseline physiological pattern relates to a baseline state of the user and the first physiological pattern relates to a first emotional or cognitive state of the user;
**characterized by**
providing a predicted indication of cognitive activation score to the user and/or to one or more other users, wherein the predicted indication is provided in relation to one or more tasks performed or to be performed by the user, and wherein the predicted indication of cognitive activation score is generated based, at least in part, on the nature of the one or more tasks using a model created from collected user data.

## Patentansprüche

1. Einrichtung, die Mittel zum Durchführen von Folgendem umfasst:
Empfangen (32) von physiologischen Messungen, die sich auf einen Benutzer beziehen, wobei die physiologischen Messungen Herzschlagmessungen umfassen;
Bestimmen (34) eines ersten physiologischen Musters auf Basis von Intervallen zwischen Spitzen der physiologischen Messungen;
Bestimmen (36) einer kognitiven Aktivierungsbewertung auf Basis eines Vergleichs zwischen dem ersten physiologischen Muster und einem grundlegenden physiologischen Muster des Benutzers, wobei sich das grundlegende physiologische Muster auf einen grundlegenden Zustand des Benutzers bezieht und sich das erste physiologische Muster auf einen ersten emotionalen oder kognitiven Zustand des Benutzers bezieht; **gekennzeichnet durch**
Bereitstellen (112) einer vorhergesagten Anzeige einer kognitiven Aktivierungsbewertung für den Benutzer und/oder für einen oder mehrere andere Benutzer, wobei die vorhergesagte Anzeige mit Bezug auf eine oder mehrere Aufgaben bereitgestellt wird, die vom Benutzer durchgeführt wurden oder durchzuführen sind, und wobei die vorhergesagte Anzeige einer kognitiven Aktivierungsbewertung mindestens teilweise auf Basis der Art der einen oder der mehreren Aufgaben unter Verwendung eines Modells erzeugt wird, das aus gesammelten Benutzerdaten erstellt wurde.

2. Einrichtung nach Anspruch 1, wobei die kognitive Aktivierungsbewertung auf Basis von einer oder mehreren Schwellwertdifferenzen zwischen dem ersten physiologischen Muster und dem grundlegenden physiologischen Muster bestimmt wird.

3. Einrichtung nach einem der vorhergehenden Ansprüche, wobei sich die Herzschlagmessungen auf Elektrokardiogrammdaten des Benutzers beziehen.

4. Einrichtung nach einem der vorhergehenden Ansprüche, wobei die kognitive Aktivierungsbewertung eine Anzeige des emotionalen oder kognitiven Zustands des Benutzers mit Bezug auf das Durchführen von einer oder mehreren Aufgaben umfasst.

5. Einrichtung nach einem der vorhergehenden Ansprüche, wobei das grundlegende physiologische Muster mindestens teilweise auf Basis von Eingaben vom Benutzer erhalten wird, wenn der Benutzer einem oder mehreren Stimuli unterzogen wird.

6. Einrichtung nach Anspruch 5, die ferner Mittel für Folgendes umfasst:
Erhalten (52) von historischen physiologischen Mustern des Benutzers;
Bestimmen (54) von Grenzen im historischen physiologischen Muster auf Basis einer Vielzahl von emotionalen oder kognitiven Zuständen; und
Anwenden (56) von Labeln für die Vielzahl von emotionalen oder kognitiven Zuständen.

7. Einrichtung nach einem der vorhergehenden Ansprüche, wobei das Bestimmen des ersten physiologischen Musters Folgendes umfasst:
Berechnen (82) von einem oder mehreren minimalen Punkten und maximalen Punkten von empfangenen physiologischen Messungen für eine erste Schwellwertzeitperiode; und
Bestimmen (84) von einer oder mehreren R-Spitzen unter Verwendung einer Spitzendetektionsfunktion,
wobei die Spitzendetektionsfunktion das Detektieren einer ersten Spitze durch Durchführen von Folgendem umfasst:
Speichern (101) von Informationen für eine erste Spitze von einem Startpunkt einer zunehmenden Schräge;
Berechnen (102) einen Dichte der ersten Spitze;
Berechnen (104) eines ersten Winkels der ersten Spitze nach dem Start einer abnehmenden Schräge;
Berechnen (105) eines Bereichs der ersten Spitze, wenn die abnehmende Schräge endet;
wenn der Startpunkt einen Abstand von einer vorherigen Spitze aufweist, der größer ist als ein Schwellwertabstand, dann:
Berechnen (107) von Ausrichtungswerten der ersten Spitze mit Bezug auf andere Spitzen; und
Bestimmen (108) auf Basis der Ausrichtungswerte eines, ob die erste Spitze eine R-Spitze ist.

8. Einrichtung nach einem der vorhergehenden Ansprüche, die ferner Mittel zum Bereitstellen der Anzeige auf einem Kalender umfasst, wobei die Anzeige von Zeitstempeln auf dem Kalender begleitet wird.

9. Einrichtung nach einem der vorhergehenden Ansprüche, die ferner Mittel zum Planen (114) von einer oder mehreren Aufgaben auf Basis der kognitiven Aktivierungsbewertung des Benutzers umfasst.

10. Einrichtung nach einem der vorhergehenden Ansprüche, die ferner eine erste Zeitperiode, bevor die kognitive Aktivierungsbewertung des Benutzers als über oder unter einem ersten Schwellwert vorhergesagt wird, Mittel zum Senden (116) eines Auslösers an den Benutzer umfasst.

11. Verfahren, das Folgendes umfasst:
Empfangen (32) von physiologischen Messungen, die sich auf einen Benutzer beziehen, wobei die physiologischen Messungen Herzschlagmessungen umfassen;
Bestimmen (34) eines ersten physiologischen Musters auf Basis von Intervallen zwischen Spitzen der physiologischen Messungen;
Bestimmen (36) einer kognitiven Aktivierungsbewertung auf Basis eines Vergleichs zwischen dem ersten physiologischen Muster und einem grundlegenden physiologischen Muster des Benutzers, wobei sich das grundlegende physiologische Muster auf einen grundlegenden Zustand des Benutzers bezieht und sich das erste physiologische Muster auf einen ersten emotionalen oder kognitiven Zustand des Benutzers bezieht; **gekennzeichnet durch**
Bereitstellen (112) einer vorhergesagten Anzeige einer kognitiven Aktivierungsbewertung für den Benutzer und/oder für einen oder mehrere andere Benutzer, wobei die vorhergesagte Anzeige mit Bezug auf eine oder mehrere Aufgaben bereitgestellt wird, die vom Benutzer durchgeführt wurden oder durchzuführen sind, und wobei die vorhergesagte Anzeige einer kognitiven Aktivierungsbewertung mindestens teilweise auf Basis der Art der einen oder der mehreren Aufgaben unter Verwendung eines Modells erzeugt wird, das aus gesammelten Benutzerdaten erstellt wurde.

12. Verfahren nach Anspruch 11, wobei die kognitive Aktivierungsbewertung auf Basis von einer oder mehreren Schwellwertdifferenzen zwischen dem ersten physiologischen Muster und dem grundlegenden physiologischen Muster bestimmt wird.

13. Computerprogramm, das Anweisungen umfasst, die, wenn das Programm von einem Computer ausgeführt wird, den Computer veranlassen, mindestens Folgendes durchzuführen:
Empfangen von physiologischen Messungen, die sich auf einen Benutzer beziehen, wobei die physiologischen Messungen Herzschlagmessungen umfassen;
Bestimmen eines ersten physiologischen Musters auf Basis von Intervallen zwischen Spitzen der physiologischen Messungen;
Bestimmen einer kognitiven Aktivierungsbewertung auf Basis eines Vergleichs zwischen dem ersten physiologischen Muster und einem grundlegenden physiologischen Muster des Benutzers, wobei sich das grundlegende physiologische Muster auf einen grundlegenden Zustand des Benutzers bezieht und sich das erste physiologische Muster auf einen ersten emotionalen oder kognitiven Zustand des Benutzers bezieht; **gekennzeichnet durch**
Bereitstellen einer vorhergesagten Anzeige einer kognitiven Aktivierungsbewertung für den Benutzer und/oder für einen oder mehrere andere Benutzer, wobei die vorhergesagte Anzeige mit Bezug auf eine oder mehrere Aufgaben bereitgestellt wird, die vom Benutzer durchgeführt wurden oder durchzuführen sind, und wobei die vorhergesagte Anzeige einer kognitiven Aktivierungsbewertung mindestens teilweise auf Basis der Art der einen oder der mehreren Aufgaben unter Verwendung eines Modells erzeugt wird, das aus gesammelten Benutzerdaten erstellt wurde.

## Revendications

1. Appareil comprenant des moyens pour :
recevoir (32) des mesures physiologiques relatives à un utilisateur, dans lequel les mesures physiologiques comprennent des mesures de rythme cardiaque ;
sur la base d'intervalles entre les pics des mesures physiologiques, déterminer (34) un premier motif physiologique ;
déterminer (36) un score d'activation cognitive sur la base d'une comparaison entre le premier motif physiologique et un motif physiologique de référence de l'utilisateur, dans lequel le motif physiologique de référence concerne un état de référence de l'utilisateur et le premier motif physiologique concerne un premier état émotionnel ou cognitif de l'utilisateur ;
**caractérisé par** l'étape suivante
fournir (112) une indication prédite d'un score d'activation cognitive à l'utilisateur et/ou à un ou plusieurs autres utilisateurs, dans lequel l'indication prédite est fournie en relation avec une ou plusieurs tâches réalisées ou à réaliser par l'utilisateur, et dans lequel l'indication prédite d'un score d'activation cognitive est générée en se basant au moins en partie sur la nature des une ou plusieurs tâches en utilisant un modèle créé à partir des données utilisateur collectées.

2. Appareil selon la revendication 1, dans lequel le score d'activation cognitive est déterminé sur la base d'une ou plusieurs différences de seuil entre le premier motif physiologique et le motif physiologique de référence.

3. Appareil selon l'une des revendications précédentes, dans lequel les mesures de rythme cardiaque se rapportent à des données d'électrocardiogramme de l'utilisateur.

4. Appareil selon l'une des revendications précédentes, dans lequel le score d'activation cognitive comprend une indication de l'état émotionnel ou cognitif de l'utilisateur en ce qui concerne la réalisation d'une ou plusieurs tâches.

5. Appareil selon l'une des revendications précédentes, dans lequel le motif physiologique de référence est obtenu en se basant au moins en partie sur des entrées de l'utilisateur lorsque l'utilisateur est soumis à un ou plusieurs stimuli.

6. Appareil selon la revendication 5, comprenant en outre des moyens pour :
obtenir (52) des motifs physiologiques historiques de l'utilisateur ;
déterminer (54) des limites dans le motif physiologique historique sur la base d'une pluralité d'états émotionnels ou cognitifs ; et
appliquer (56) des étiquettes pour la pluralité d'états émotionnels ou cognitifs.

7. Appareil selon l'une des revendications précédentes, dans lequel la détermination du premier motif physiologique comprend ce qui suit :
calculer (82) un ou plusieurs points minimaux et points maximaux de mesures physiologiques reçues pendant une première période de temps seuil ; et
déterminer (84) un ou plusieurs pics R à l'aide d'une fonction de détection de pic,
dans lequel la fonction de détection de pic comprend la détection d'un premier pic :
en stockant (101) des informations pour un premier pic à partir d'un point de départ d'une pente ascendante ;
en calculant (102) une densité du premier pic ;
en calculant (104) un premier angle du premier pic après le début d'une pente descendante ;
en calculant (105) une superficie du premier pic lorsque la pente descendante se termine ;
si le point de départ est à distance d'un pic précédent qui est supérieure à une distance seuil, alors :
en calculant (107) des valeurs d'alignement du premier pic en ce qui concerne les autres pics ; et
en déterminant (108) un si le premier pic est un pic R sur la base des valeurs d'alignement.

8. Appareil selon l'une des revendications précédentes, comprenant en outre des moyens pour fournir l'indication sur un calendrier, dans lequel l'indication est accompagnée d'horodatages sur le calendrier.

9. Appareil selon l'une des revendications précédentes, comprenant en outre des moyens pour planifier (114) une ou plusieurs tâches sur la base du score d'activation cognitive de l'utilisateur.

10. Appareil selon l'une des revendications précédentes, comprenant en outre des moyens pour envoyer (116) un déclencheur à l'utilisateur dans une première période de temps avant que le score d'activation cognitive de l'utilisateur ne soit prédit comme étant au-dessus d'un premier seuil ou en dessous de celui-ci.

11. Procédé comprenant les étapes suivantes :
recevoir (32) des mesures physiologiques relatives à un utilisateur, dans lequel les mesures physiologiques comprennent des mesures de rythme cardiaque ;
sur la base d'intervalles entre les pics des mesures physiologiques, déterminer (34) un premier motif physiologique ;
déterminer (36) un score d'activation cognitive sur la base d'une comparaison entre le premier motif physiologique et un motif physiologique de référence de l'utilisateur, dans lequel le motif physiologique de référence concerne un état de référence de l'utilisateur et le premier motif physiologique concerne un premier état émotionnel ou cognitif de l'utilisateur ;
**caractérisé par** l'étape suivante
fournir (112) une indication prédite d'un score d'activation cognitive à l'utilisateur et/ou à un ou plusieurs autres utilisateurs, dans lequel l'indication prédite est fournie en relation avec une ou plusieurs tâches réalisées ou à réaliser par l'utilisateur, et dans lequel l'indication prédite d'un score d'activation cognitive est générée en se basant au moins en partie sur la nature des une ou plusieurs tâches en utilisant un modèle créé à partir des données utilisateur collectées.

12. Procédé selon la revendication 11, dans lequel le score d'activation cognitive est déterminé sur la base d'une ou plusieurs différences de seuil entre le premier motif physiologique et le motif physiologique de référence.

13. Programme informatique comprenant des instructions qui, lorsque le programme est exécuté par un ordinateur, amènent l'ordinateur à au moins :
recevoir des mesures physiologiques relatives à un utilisateur, dans lequel les mesures physiologiques comprennent des mesures de rythme cardiaque ;
sur la base d'intervalles entre les pics des mesures physiologiques, déterminer un premier motif physiologique ;
déterminer un score d'activation cognitive sur la base d'une comparaison entre le premier motif physiologique et un motif physiologique de référence de l'utilisateur, dans lequel le motif physiologique de référence concerne un état de référence de l'utilisateur et le premier motif physiologique concerne un premier état émotionnel ou cognitif de l'utilisateur ;
**caractérisé par** l'étape suivante
fournir une indication prédite d'un score d'activation cognitive à l'utilisateur et/ou à un ou plusieurs autres utilisateurs, dans lequel l'indication prédite est fournie en relation avec une ou plusieurs tâches réalisées ou à réaliser par l'utilisateur, et dans lequel l'indication prédite d'un score d'activation cognitive est générée en se basant au moins en partie sur la nature des une ou plusieurs tâches en utilisant un modèle créé à partir des données utilisateur collectées.
